# EUROPÄISCHE PATENTSCHRIFT

(11) **EP 2 114 501 B1**
(45) Veröffentlichungstag und Bekanntmachung des Hinweises auf die Patenterteilung: **18.11.2015**
(21) Anmeldenummer: 08702573.0
(22) Anmeldetag: 05.02.2008
(51) Int. Cl.: A61M 16/20, A61M 16/00

(54) **Steuerventil für Beatmungsgeräte**
Control valve for respiratory devices
Valve de commande pour appareil respiratoire

(30) Priorität: 05.02.2007 CH 1842007
(43) Veröffentlichungstag der Anmeldung: 11.11.2009
(73) Patentinhaber: Imtmedical AG, 9470 Buchs SG (CH)
(72) Erfinder: FRIBERG, Harri, 9493 Mauren (LI); DÄSCHER, Jakob, CH-9470 Buchs (CH); WIRTH, Michael, 7477 Trübbach (CH); KRAMER, Felix, 9000 St. Gallen (CH)
(74) Vertreter: Rosenich, Paul
(86) Internationale Anmeldenummer: PCT/IB2008/050415
(87) Internationale Veröffentlichungsnummer: WO 2008/096316

(56) Entgegenhaltungen:
- EP-A- 0 621 052
- WO-A-92/11054
- WO-A-99/47839
- DE-U1- 29 922 220
- GB-A- 288 403
- GB-A- 924 377
- GB-A- 2 215 218
- US-A- 3 159 179
- US-A- 6 148 816

## Beschreibung

Die Erfindung betrifft ein Ventil für die Durchfluss-Steuerung und -Regulierung eines Beatmungsgerätes, wobei das Ventil ein Gehäuse und einen darin um seine Längsachse verdrehbar angeordneten Drehschieber aufweist und wobei durch Verdrehen des Drehschiebers wenigstens eine Durchtrittsöffnung ganz oder teilweise verschliessbar ist und der Drehschieber eine mit einer korrespondierend ausgebildeten Stützfläche des Gehäuses zusammenwirkende, kegelförmige Dichtfläche aufweist und die Durchtrittsöffnung im Bereich der Dichtfläche angeordnet ist.

Beatmungsgeräte werden sowohl im stationären (z.B. Klinik- oder Heimbereich) als auch im mobilen Bereich (z.B. Rettungsdienst) eingesetzt. Dabei ist es sehr wichtig, dass diese Geräte zuverlässig und störungsfrei arbeiten. Um Störungen der Umgebung beim Betreiben von solchen Geräten zu vermeiden, müssen diese Geräte im Betrieb auch möglichst leise sein.

Aus der US 6,615,831 ist ein Beatmungsgerät mit 3/2-Weg-Ventil bekannt. Das Ventil weist einen mittels einer Magnetspule axial verschiebbaren Steuerschieber auf, der in seinen Endstellungen Durchtrittsöffnungen für die von einem Druckgenerator stammende Beatmungsluft öffnet, bzw. diese verschliesst und gleichzeitig separate Durchtrittsöffnungen öffnet, um einen minimalen Luftverbrauch für den Druckgenerator zu ermöglichen. Dieses Ventil ist jedoch sehr stark lageabhängig und, infolge des für die axiale Beweglichkeit des Steuerschiebers erforderlichen Radialspiels, im geschlossenen Zustand auch undicht.

Die EP 1 177 810 B1 zeigt ein Ventil mit einem um seine Längsachse verdrehbaren Drehschieber. Dieser weist an seinem Umfang wenigstens einen Längsschlitz auf, der mit entsprechenden Öffnungen des den Schieber umgebenden Gehäuseteils zur Deckung gebracht werden kann. An der freien Stirnseite des Drehschiebers ist ausserdem eine Steuerkurve angebracht, durch welche die Öffnungen auch nur teilweise freigelegt werden können. Dieses Ventil weist ebenfalls den Nachteil auf, dass es in geschlossenem Zustand nie völlig dicht ist. Um die Dynamik des Ventils zu gewährleisten, muss der Drehschieber mit einem gewissen Radialspiel in das Gehäuseteil eingepasst werden. Durch den dabei entstehenden Ringspalt kann ein Teil der Atemluft entweichen und somit die gewünschte Dosierung negativ beeinflussen.

Aus der GB 288 403 ist ein Ventil, bzw. ein Schieber für Gase und andere Fluids bekannt. Dieses weist ein Gehäuse mit einem Kegelsitz und einen kegelstumpfförmigen, von aussen durch Verdrehen betätigbaren Ventilkörper auf. Beim Verdrehen des Ventilkörpers werden Durchtrittsöffnungen im Kegelsitz des Gehäuses mit Öffnungen am Ventilkörper zu Deckung gebracht und somit ein Durchströmen des Mediums durch das Ventil ermöglicht. Durch die grossflächige Auflage des Ventilkörpers im Kegelsitz entsteht jedoch ein relativ hoher Reibungswiderstand, der sich auf die Betätigung hindernd auswirkt und eine dynamische Steuerung des Ventils verhindert.

Der Erfindung liegt daher die Aufgabe zugrunde, ein Beatmungsgerät, bzw. ein Ventil für ein Beatmungsgerät zu schaffen, das betriebssicher ist, einen geringen Durchflusswiderstand aufweist, sich durch eine hohe Dynamik auszeichnet und in geschlossenem Zustand eine hohe Dichtheit des Ventils ermöglicht.

Gemäss der Erfindung wird dies dadurch erreicht, dass der Drehschieber in axialer Richtung über eine zentrale Punktauflage, vorzugsweise eine in der Längsmittelachse angeordnete Kugel auf dem Gehäuse abgestützt ist. Eine Punktauflage zwischen dem Drehschieber und dem Gehäuse ergibt geringe Reibungsverluste und ermöglicht dadurch mittels kleinen Stellkräften und -Momenten eine hohe Dynamik des Ventils. Durch die kegelförmige Dichtfläche wird der Drehschieber beim Verschliessen des Ventils durch den Überdruck der Beatmungs-, bzw. Ausatmungsluft gegen die Dichtfläche gepresst. Leckverluste werden dadurch praktisch vermieden. Abnützungen des Drehschiebers und der korrespondierenden Stützfläche infolge Verschleiss werden automatisch kompensiert.

Der Kegel der Dichtfläche ist vorteilhaft sich entgegen der Durchflussrichtung verjüngend ausgebildet. Beim Anströmen des Kegels durch die Beatmungs-, bzw. Ausatmungsluft entsteht dadurch eine gleichmässige Verteilung des Luftstroms im Ventil und somit ein relativ geringer Durchflusswiderstand.

Der Kegelwinkel der Dichtfläche beträgt zweckmässigerweise zwischen 60° und 120°, vorzugsweise etwa 90°. Durch diesen Winkelbereich wird eine relativ kurze und kompakte Bauweise des Ventils ermöglicht. Der genannte Winkelbereich ergibt auch günstige Strömungsverhältnisse und weitgehende Vermeidung von Turbulenzen.

Die Durchtrittsöffnungen sind zweckmässigerweise so ausgebildet, dass sich der Durchflussquerschnitt beim Verdrehen des Drehschiebers in Schliessrichtung progressiv verkleinert, und/oder dass mehrere Durchtrittsöffnungen vorgesehen sind.. Zum Ende des Schliessvorganges hin besteht nur noch ein kleiner Durchgangsquerschnitt, sodass beim völligen Schliessen des Ventils Druckschläge im System weitgehend vermieden werden. Bei mehreren Bohrungen können diese in der Grösse unterschiedlich sein, wobei die Grösse der Bohrungen gegen Ende des Schliessvorganges kleiner wird. Der gleiche Effekt kann dadurch erzielt werden, dass in radialer Richtung mehrere Bohrung nebeneinander angeordnet werden und die Anzahl der radial nebeneinander angeordneten Bohrungen gegen das Ende des Schliessvorganges abnimmt.

Die Durchtrittsöffnungen sind vorteilhaft in Schliessrichtung des Drehschiebers sich keilförmig verjüngend ausgebildet. Durch die Form des Keils kann bewirkt werden, dass sich der verbleibende Durchflussquerschnitt beim Schliessen des Ventils schneller oder langsamer verkleinert, bzw. vergrössert. Die Ränder der keilförmigen Durchtrittsöffnung sind vorzugsweise gebogen ausgebildet.

Um eine hohe Dynamik und eine gute Abdichtung des Ventils in geschlossenem Zustand zu erreichen, ist es zweckmässig, dass sich der Kegelradius der miteinander zusammenwirkenden Dichtflächen des Gehäuses und/oder des Drehschiebers) entgegen der Schliessrichtung des Ventils über Teile des Umfanges konstant vergrössert. Die aneinander liegenden Dichtflächen des Drehschiebers und des Gehäuses sind also keine genauen Kegelflächen, sondern weichen gemäss dieser besonderen Ausgestaltung geringfügig von einer Kegelfläche ab. Im geschlossenen Zustand des Ventils stützen sich die Dichtflächen flächig aufeinander ab und ergeben eine sehr hohe Abdichtung. Beim Öffnen des Ventils entsteht ein Dichtspalt, der den Durchtrittsquerschnitt zusätzlich vergrössert und somit einen höheren Durchfluss durch das Ventil ermöglicht.

Eine weitere vorteilhafte Ausführung besteht darin, dass die Durchtrittsöffnungen des Gehäuses auf der dem Drehschieber gegenüberliegenden Seite von einem Wulst umgeben sind, wobei die Randhöhe des Wulstes gegen die Schliessstellung des Ventils hin zunehmend ist. Dabei entsteht bei geschlossenem Zustand des Ventils ebenfalls eine flächige Abdichtung. Beim Öffnen des Ventils hebt der Drehschieber vom Wulst ab und es entsteht ebenfalls ein zusätzlicher Dichtspalt.

Das Steuerventil wird vorteilhaft über ein Stellglied, vorzugsweise über einen Schrittmotor proportional angesteuert. Somit gibt es im Betrieb des Ventils nicht nur zwei Endstellungen des Ventils, nämlich "offen" oder "zu" , sondern es können je nach Bedarf beliebige Zwischenstellungen angesteuert werden.

Zwischen dem Drehschieber und dem Stellglied ist vorteilhaft eine lösbare, elastische Kupplung angeordnet. Diese Kupplung ermöglicht eine spielfreie Übertragung der Rotationsbewegung vom Stellglied auf den Drehschieber. Gleichzeitig kann ein allfälliger Achsversatz ausgeglichen werden.

Für eine automatische Rückstellung in seine Ausgangslage ist der Drehschieber zweckmässigerweise gegen die Rückstellkraft wenigstens einer Torsionsfeder verdrehbar. Im stromlosen Zustand des Drehantriebs besteht somit eine definierte Ausgangslage. Bei einem Stromausfall ist bzw. geht das Ventil somit vorzugsweise automatisch in voll geöffneter Stellung, sodass die natürliche Atmung des Patienten nicht behindert wird.

Das erfindungsgemässe Ventil wird vorteilhaft in einem automatischen Beatmungsgerät zur künstlichen Beatmung, bzw. Unterstützung der Beatmung eines Patienten, mit einem Lufteingang, einer Luftquelle wenigstens einem proportional regulierbaren Steuerventil sowie Sensoren zur Messung des Drucks und des Durchflusses zur Ansteuerung des Ventils verwendet.

Die Luftquelle in einem derartigen Beatmungsgerät ist zweckmässigerweise als ein über einen gesamten Atemzyklus mit konstanter Drehzahl rotierendes Gebläse ausgebildet. Ein solches Gebläse zeichnet sich durch eine hohe Laufruhe sowie einen geringen Geräuschpegel aus. Die Regulierung der Beatmung erfolgt dabei durch die Ansteuerung der Ventile.

Weitere Ausbildungen der Erfindung sind in den Figuren und in den abhängigen Patentansprüchen angegeben.

Die Bezugszeichenliste ist, so wie die Anspruchsfassung, Bestandteil der Offenbarung.

Die Figuren werden zusammenhängend und übergreifend beschrieben. Gleiche Bezugszeichen bedeuten gleiche Bauteile, Bezugszeichen mit unterschiedlichen Indizes geben funktionsgleiche oder ähnliche Bauteile an.

Anhand von Figuren wird die Erfindung symbolisch und beispielhaft näher erläutert.

Es zeigen dabei:
- Fig. 1: ein erfindungsgemässes Beatmungsgerät, schematisch dargestellt,
- Fig. 2: eine erste Ausführungsform des erfindungsgemässen Ventils mit kegelförmigem Ventilsitz, im Längsschnitt dargestellt,
- Fig. 3: einen Querschnitt durch das in Fig. 2 dargestellte Ventil, entlang der Schnittlinie A-A, In geöffnetem Zustand,
- Fig. 4: einen Querschnitt analog Fig. 3, in geschlossenem Zustand,
- Fig. 5: einen Querschnitt entlang der Schnittlinie C - C in Fig. 10 durch eine weitere Ausführung eines erfindungsgemässen Ventils, Im geöffneten Zustand,
- Fig. 6: das Ventil gemäss Fig. 5, in geschlossenem Zustand und
- Fig. 7: einen Längsschnitt durch das aus Fig. 5 und 6 ersichtliche Ventil.

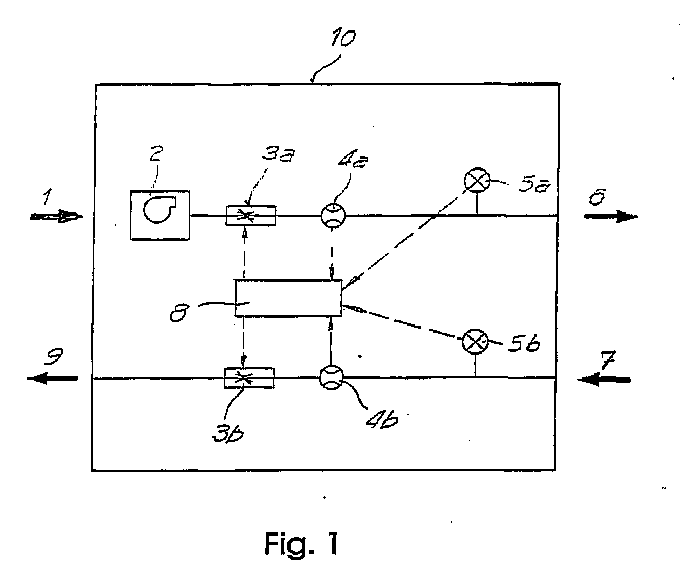

Das in Fig. 1 schematisch dargestellte, zum Schutz mit einer Verschalung 10 versehene Beatmungsgerät weist einen Lufteingang 1 und eine Luftquelle 2 auf. Die Luftquelle 2 ist vorzugsweise als Gebläse oder Kompressor ausgebildet. Als Luftquelle 2 kann aber auch ein interner oder externer Druckspeicher oder eine externe Druckluftversorgung verwendet werden. Im oberen, der Einatmung eines Patienten dienenden Strangs ist ein regulierbares Einatmungs-Steuerventil 3a, ein Durchluss-Sensor 4a für die Einatmung und ein Druck-Sensor 5a für die Einatmung angeordnet Am Ende dieses Strangs führt ein Einatmungsschlauch 6 zum Patienten. Ein Ausatmungsschlauch 7 führt vom Patienten zurück in die Verschalung 10. In diesem unteren, die Ausatmung steuernden Strang ist ebenfalls ein Druck-Sensor 5b für die Ausatmung, ein Durchfluss-Sensor 4b für die Ausatmung und ein regulierbares Ausatmungs-Steuerventil 3b angeordnet. Die Messwerte der Durchfluss- und Druck-Sensoren werden laufend einer Steuerung 8 zugeführt, wo sie zur Regelung der Steuerventile 3a, 3b für die Einatmung, bzw. Ausatmung des Patienten dienen. Schliesslich gelangt die ausgeatmete Atemluft über einen Luftaustritt 9 ins Freie.

Das Beatmungsgerät kann direkt an einem externen Stromnetz oder durch eine interne oder extreme Batterie betrieben werden.

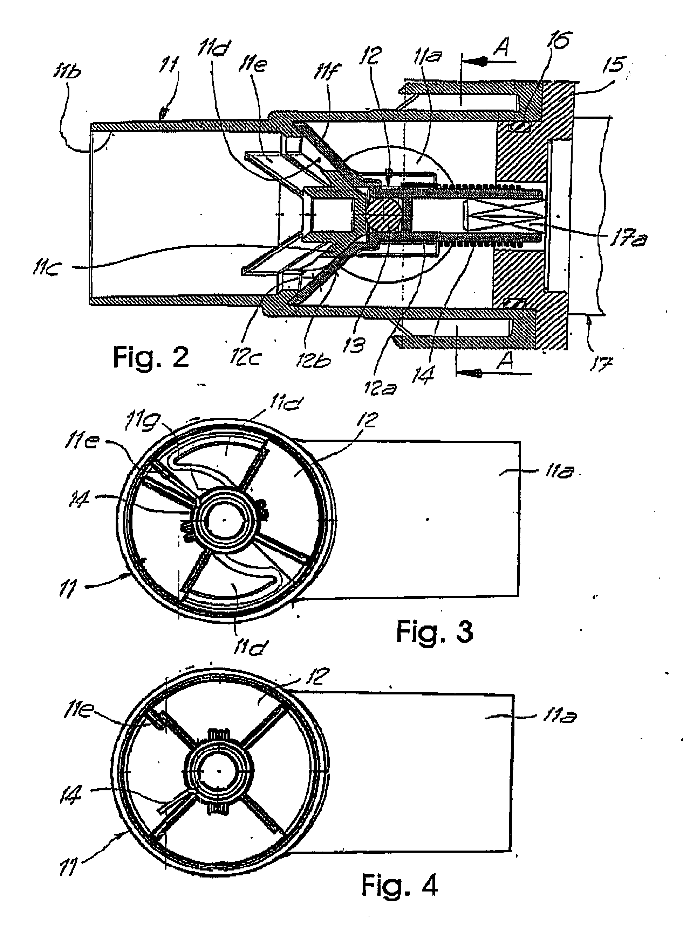

Die aus den Fig. 3 bis 4 ersichtliche Ausführung eines erfindungsgemässen Steuerventils besteht aus einem Gehäuse 11 mit einem darin drehbar gelagerten Drehschieber 12. Das Gehäuse 11 weist einen seitlichen Eintrittstutzen 11a und einen In axialer Richtung an den Drehschieber 12 anschliessenden Austrittsstutzen 11b auf. Zwischen dem Eintrittstutzen 11a und dem Austrittstutzen 11b befindet sich eine Zwischenwand 11c. Die Zwischenwand 11c ist kegelförmig ausgebildet und schliesst einen Kegelwinkel von ca. 90° ein. Die Zwischenwand 11c ist mit keilförmig ausgebildeten Durchtrittsöffnungen 11d versehen. Der Drehschieber 12 besteht aus einem rohrförmigen Schaft 12a und einem damit verbundenen Trichter 12b. Die Innenseite des Trichters 12b bildet zusammen mit einer Stützfläche 11f an der Zwischenwand 11 c des Gehäuses 11 eine Dichtfläche 12c, welche in dem in Fig. 4 dargestellten geschlossenen Zustand des Ventils die Durchtrittsöffnungen 11d verschliesst.

Wie aus Fig. 2 hervorgeht, ist der Drehschieber 12 in axialer Richtung über eine Kugel 13 auf dem Gehäuse 11 zentrisch abgestützt. Die Kugel 13 ergibt an beiden Teilen 11, 12 eine zentrale Punktauflage im Bereich der Längsmittelachse und ermöglicht somit eine sehr geringe Reibung.

Eine Torsionsfeder 14, welche den Schaft 12a des Drehschiebers 12 umgibt, dient dazu, den Drehschieber 12 im stromlosen Zustand des Ventils in einer definierten Ausgangslage zu halten. (z.B. "offen" oder "zu"). Zudem unterstützt die Torsionsfeder 14 das Dichthalten des Drehschiebers 12, indem der Trichter 12b in axialer Richtung gegen die Zwischenwand 11c gedrückt wird. Ein Deckel 15 ist auf der dem Austrittstutzen 11 b gegenüberliegenden Seite mit dem Gehäuse 11 verbunden. Ein vorzugsweise als O-Ring ausgebildeter Dichtring 16 dient zur Abdichtung des Deckels 15.

Am Deckel 15 ist ein als Stellantrieb dienender Schrittmotor 17 befestigt. Der Schrittmotor 17 dient dem Drehantrieb des Drehschiebers 12 mittels einer Antriebswelle 17a. Beim Schliessen des Ventils entsteht hinter dem Ventil ein Überdruck, welcher den Trichter 12b, bzw. die Dichtfläche 12c des Drehschiebers 12, axial gegen die Stützfläche 11f des Gehäuses 11 presst. Dadurch entstehen praktisch keine Leckverluste. Dies ermöglicht das Betreiben einer beispielsweise als Gebläse ausgebildeten Luftquelle mit konstanter Drehzahl über einen kompletten Atemzyklus, bestehend aus Einatmung und Ausatmung. Die gesamte Dynamik des Atemzyklusses kann demnach alleine mit den Steuerventilen erreicht werden. In Längsrichtung ausgerichtete Leitflügel 11 e sollen eine Verwirbelung der Strömung im Ventil verhindern. Diese Leitflügel sind jedoch fakultativ und können somit auch entfallen.

Wie aus Fig. 3 ersichtlich ist, sind die Durchtrittsöffnungen 11d keilförmig mit gebogenen Rändern ausgebildet. Dies ermöglicht eine bestimmte, z.B. progressive Schliess-, bzw. -Öffnungs-Charakteristik des Ventils. Insbesondere können damit auch Druckspitzen abgebaut, bzw. verhindert werden. Zum Schliessen des Ventils wird der Drehschieber 12 durch die Kraft der Torsionsfeder 14 in die In Fig. 4 dargestellte geschlossene Position verdreht. Je nach Luftbedarf können die Durchtrittsöffnungen 11d durch einen geringeren Schwenkwinkel des Drehschiebers 12 auch nur teilweise geöffnet werden.

Die Durchtrittsöffnungen 11d sind von einem Wulst 11g umgeben. Die Randhöhe des Wulstes 11g ist vorzugsweise In Schliessrichtung zunehmend. Somit wird eine Auflaufschräge für den Drehschieber 12 gebildet Dadurch wird in geschlossenem Zustand des Ventils eine besonders gute Abdichtung erreicht Beim Öffnen des Ventils kann der Drehschieber 12 vom Wulst 11g abheben, sodass ein zusätzlicher Dichtspalt geöffnet und der Durchsatz des Ventils vergrössert wird.

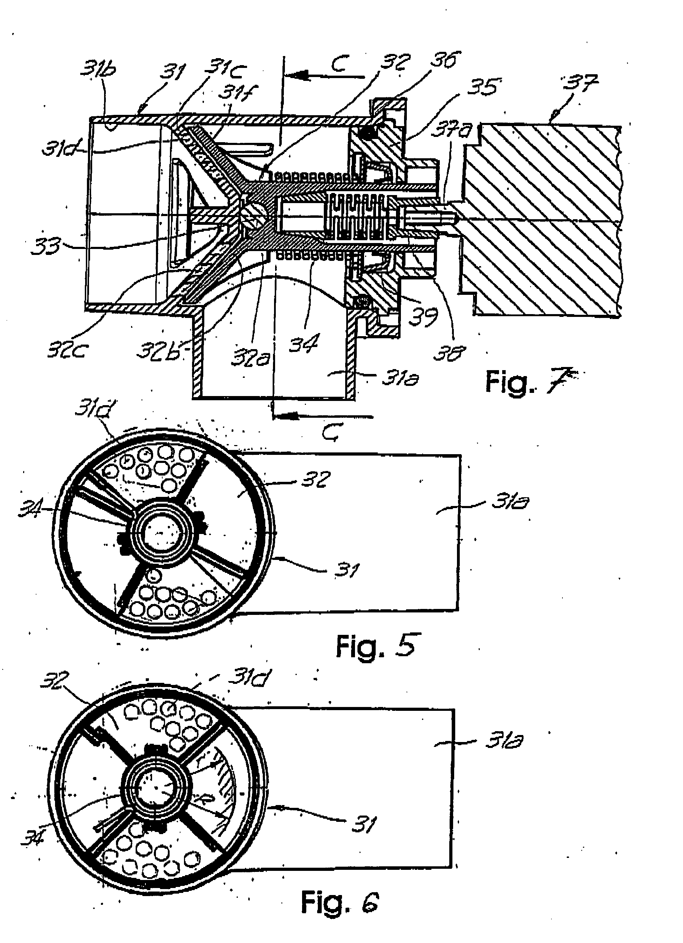

Das aus den Fig. 5 bis 7 ersichtliche Ventil entspricht funktionell im wesentlichen der aus den Fig. 2 bis 4 gezeigten Ausführung und besteht ebenfalls aus einem Gehäuse 31 und einem darin drehbar gelagerten Drehschieber 32. Seitlich am Gehäuse 31 ist ein Eintrittsstutzen 31 angeformt. Ein Austrittsstutzen 31 b ist koaxial zur Längsachse des Drehschiebers 32 angeordnet. Zwischen dem Eintrittsstutzen 31 a und dem Austrittsstutzen 31 b befindet sich eine kegelförmige Zwischenwand 31c. Die Zwischenwand 31c bildet eine Stützfläche 31f für den Drehschieber 32. Der Drehschieber 32 weist einen im wesentlichen zylindrischen Schaft 32a und einen damit verbundenen Trichter 32b auf. Im Bereich des Trichters 32b sind Dichtflächen 32c angeordnet, welche zusammen mit den Stützflächen 31f eine flächige Abdichtung bilden.

Der Drehschieber 32 ist über eine zentral angeordnete Kugel 33 punktförmig auf dem Gehäuse 31 abgestützt und ist somit gegen einen sehr geringen Widerstand verdrehbar. Eine Torsionsfeder 34 hält den Drehschieber 32 In der in Fig. 5 dargestellten Ausgangsstellung. Dabei sind Durchtrittsöffnungen 31d des Gehäuses 31 voll geöffnet und ermöglichen Durchströmen des Ventils. Im Unterschied zu der aus Fig. 2 bis 4 ersichtlichen Ausführung, welche auf beiden Seiten nur eine einzige, keilförmige Durchtrittsöffnung (11d) aufweist, sind bei dieser Ausführung die Durchtrittsöffnungen 31 d als eine Vielzahl einzelner Bohrungen ausgebildet. Dabei sind die Bohrungen so angeordnet, dass sich der Durchflussquerschnitt ebenfalls in Schliessrichtung progressiv verkleinert.

Das rückwärtige Ende des Ventils wird durch einen Deckel 35 abgeschlossen. Zur Abdichtung zwischen dem Gehäuse 31 und und dem Deckel 35 ist ein Dichtring 36 vorgesehen. Eine mit dem Schaft 32a des Drehschiebers 32 zusammenwirkende Wellendichtung 39 verhindert Leckverluste der Beatmungsluft und gleichzeitig das Eindringen von Fremdstoffen.

Der Drehschieber 32 wird ebenfalls über einen beispielsweise als Schrittmotor 37 ausgebildet Stellantrieb angetrieben. Die Antriebswelle 37a des Schrittmotors 37 ist dabei Ober eine lösbare, elastische Kupplung 38 mit dem Drehschieber 32 verbunden. Die Flexibilität der Kupplung 38 ergibt wenigstens drei vorteilhafte Effekte:
1. die Rotation wird vom Schrittmotor 37 spielfrei auf den Drehschieber 32 übertragen.
2. ein allfälliger Achsversatz zwischen dem Schrittmotor 37 und dem Drehschieber wird ausgeglichen und
3. die Kupplung kann eine zusätzliche axiale Kraft aufbringen, die dazu führt, dass das drehschieberseitige Ende der Kupplung 38 stets satt in der Aufnahme der Drehschiebers 32 anliegt.

Im Unterschied zu der aus Fig. 2 bis 4 ersichtlichen Ausführung, bei der die Stützfläche 11f und die Dichtfläche 12c als exakter Kegel ausgebildet sind, weisen die Dichtfläche 32c und die Stützfläche 31f über den Umfang einen variablen Kegelradius r / R auf, der sich entgegen der Schliessrichtung kontinuierlich vergrössert. Der Steigungswinkel der Radiusänderung beträgt vorzugsweise ca. 3°. Dadurch kann erreicht werden, dass in geschlossenem Zustand des Ventils die Kugel 33 von der Punktauflage abgehoben und die Dichtflächen voll axial gegeneinander gepresst werden. Dadurch wird eine hohe Abdichtung des Ventils in geschlossenem Zustand erreicht.

Sowohl das Gehäuse 11, 31 als auch der Drehschieber 12, 32 bestehen vorzugsweise aus Kunststoff. Kunststoffe weisen ein relativ geringes spezifisches Gewicht auf und ergeben somit ein geringes Eigengewicht der beweglichen Teile, insbesondere des Drehschiebers12, 32. Dies wiederum ermöglich eine hohe Dynamik der Bewegung dieser Teile. Ein weitere Vorteil der Kunststoffe besteht darin, dass sie dauerhaft und sehr gut sterilisierbar sind.

### Bezugszeichenliste

- 1: Lufteingang
- 2: Luftquelle
- 3a: Einatmungs-Steuerventil
- 3b: Ausatmungs-Steuerventil
- 4a: Durchfluss-Sensor für Beatmung
- 4b: Durchfluss-Sensor für Ausatmung
- 5a: Druck-Sensor für Beatmung
- 5b: Druck-Sensor für Ausatmung
- 6: Beatmungsschlauch
- 7: Ausatmungsschlauch
- 8: Steuerung
- 9: Luftaustritt
- 10: Verschalung

- 11: Gehäuse
- 11a: Eintrittstutzen
- 11b: Austrittstutzen
- 11c: Zwischenwand
- 11d: Durchtrittsöffnungen
- 11e: Leitflügel
- 11f: Stützfläche
- 11g: Wulst
- 12: Drehschieber
- 12a: Schaft
- 12b: Trichter
- 12c: Dichtfläche
- 13: Kugel
- 14: Torsionsfeder
- 15: Deckel
- 16: Dichtring
- 17: Schrittmotor
- 17a: Antriebswelle

- 31: Gehäuse
- 31a: Eintrittstutzen
- 31b: Austrittsstutzen
- 31c: Zwischenwand
- 31d: Durchtrittsöffnungen
- 31f: Stützfläche
- 32: Drehschieber
- 32a: Schaft
- 32b: Trichter
- 32c: Dichtfläche
- 33: Kugel
- 34: Torsionsfeder
- 35: Deckel
- 36: Dichtring
- 37: Schrittmotor
- 37a: Antriebswelle
- 38: Kupplung
- 39: Wellendichtring

## Patentansprüche

1. Ventil für die Durchfluss-Steuerung und -Regulierung eines Beatmungsgerätes (1), wobei das Ventil ein Gehäuse (11) und einen darin um seine Längsachse verdrehbar angeordneten Drehschieber (12) aufweist und wobei durch Verdrehen des Drehschiebers (12) wenigstens eine Durchtrittsöffnung (11d) ganz oder teilweise verschliessbar ist, wobei der Drehschieber (12) eine mit einer korrespondierend ausgebildeten Stützfläche (11f) des Gehäuses (11) zusammenwirkende, kegelförmige Dichtfläche (12c) aufweist und die Durchtrittsöffnung (11d) im Bereich der Dichtfläche (11f) angeordnet Ist, **dadurch gekennzeichnet, dass** der Drehschieber (12) in axialer Richtung Ober eine zentrale Punktauflage, vorzugsweise eine in der Längsmittelachse angeordnete Kugel (13), auf dem Gehäuse (11) abgestützt ist.

2. Ventil nach Anspruch 1, **dadurch gekennzeichnet, dass** der Kegel der Dichttläche (12c) sich entgegen einer Durchflussrichtung verjüngend ausgebildet ist.

3. Ventil nach Anspruch 1 oder 2, **dadurch gekennzeichnet, dass** der Kegelwinkel der Dichtfläche zwischen 60° und 120°, vorzugsweise etwa 90° beträgt.

4. Ventil nach einem der vorhergehenden Ansprüche, **dadurch gekennzeichnet, dass** die Durchtrittsöffnung (11 d) so ausgebildet ist, dass sich der Durchflussquerschnitt beim Verdrehen des Drehschiebers (12) in Schliessrichtung progressiv verkleinert, und/oder dass mehrere Durchtrittsöffnungen vorgesehen sind.

5. Ventil nach Anspruch 4, **dadurch gekennzeichnet, dass** die Durchtrittsöffnung bzw. Durchtrittsöffnungen (11d) in Schliessrichtung des Drehschiebers (12) sich keilförmig verjüngend ausgebildet Ist, bzw. sind.

6. Ventil nach einem der vorhergehenden Ansprüche, **dadurch gekennzeichnet, dass** sich der Kegelradius (r/R) der miteinander zusammenwirkenden Dichtflächen des Gehäuses (31) und/oder des Drehschiebers (32) entgegen der Schliessrichtung des Ventils über Teile des Umfanges kontinuierlich vergrössert.

7. Ventil nach einem der vorhergehenden Ansprüche, **dadurch gekennzeichnet, dass** die Durchtrittsöffnungen (11 d) des Gehäuses (11) auf der dem Drehschieber (12) gegenüberliegenden Seite von einem Wulst (11g) umgeben sind, wobei die Randhöhe des Wulstes (11 g) gegen die Schliessstellung des Ventils hin zunehmend ist.

8. Ventil nach einem der vorhergehenden Ansprüche, **dadurch gekennzeichnet, dass** das der Drehschieber (12) über ein Stellglied, vorzugsweise über einen Schrittmotor (17, 37) proportional angesteuert wird.

9. Ventil nach Anspruch 8, **dadurch gekennzeichnet, dass** zwischen dem Drehschieber (12) und dem Stellglied eine lösbare, elastische Kupplung (38) angeordnet ist.

10. Ventil nach einem der vorhergehenden Ansprüche, **dadurch gekennzeichnet, dass** der Drehschieber (12,32) gegen die Rückstellkraft wenigstens einer Torsionsfeder (14, 34)) verdrehbar ist.

11. Beatmungsgerät zur künstlichen Beatmung, bzw. Unterstützung der Beatmung eines Patienten, mit einem Lufteingang (1), einer Luftquelle (2), wenigstens einem proportional regulierbaren Steuerventil (3a, 3b) sowie Sensoren zur Messung des Drucks (5a, 5b) und des Durchflusses (4a, 4b) zur Ansteuerung des Steuerventils (3a, 3b), **dadurch gekennzeichnet, dass** das Steuerventil ein Ventil gemäss einem der Ansprüche 1 bis 10 ist.

12. Beatmungsgerät nach Anspruch 11, **dadurch gekennzeichnet, dass** die Luftquelle (2) als ein über einen gesamten Atemzyklus mit konstanter Drehzahl rotierendes Gebläse ausgebildet ist.

## Claims

1. A valve for the flow control and regulation of a ventilation apparatus (1), wherein the valve has a casing (11) in which a rotary vane (12) is arranged so that it can rotate about its longitudinal axis, and wherein at least one opening (11d) can be partially or fully closed by rotating the rotary vane (12), wherein the rotary vane (12) has a cone-shaped sealing surface (12c) cooperating with a correspondingly shaped cone-shaped supporting surface (11f) of the casing (11) and the opening (11d) is located in the area of the sealing surface (11f), **characterised in that** the rotary vane (12) is supported in axial direction on the casing (11) via a central point support, preferably a ball (13) arranged along the longitudinal centre axis.

2. The valve according to claim 1, **characterised in that** the cone of the sealing surface (12c) is arranged so that it tapers contrary to a flow direction.

3. The valve according to claims 1 or 2, **characterised in that** the angle of the cone of the sealing surface is between 60° and 120°, preferably approximately 90°.

4. The valve according to any of the preceding claims, **characterised in that** the opening (11d) is shaped so that the flow cross-section gets progressively smaller in closing direction during rotation of the rotary vane (12), and/or **in that** a number of openings are provided.

5. The valve according to claim 4, **characterised in that** the one or more openings (11d) are wedge-shaped and tapering in closing direction of the rotary vane (12).

6. The valve according to any of the preceding claims, **characterised in that** the radius of the cone (r/R) of the cooperating sealing surfaces of the casing (31) and/or of the rotary vane (32) continuously increases over parts of the circumference, contrary to the closing direction of the valve.

7. The valve according to any of the preceding claims, **characterised in that** the openings (11d) of the casing (11) are surrounded by a bead (11g) on the side opposite the rotary vane (12), wherein the rim height of the bead (11g) gradually increases towards the closed position of the valve.

8. The valve according to any of the preceding claims, **characterised in that** the rotary vane (12) is proportionally activated by an actuator, preferably by a stepping motor (17, 37).

9. The valve according to claim 8, **characterised in that** a detachable elastic coupling (38) is arranged between the rotary vane (12) and the actuator.

10. The valve according to any of the preceding claims, **characterised in that** the rotary vane (12, 32) can be rotated against the reset force of at least one torsion spring (14, 34).

11. A ventilation apparatus for artificial respiration, or supporting the respiration of a patient, with an air intake (1), an air source (2), at least one proportionally adjustable control valve (3a, 3b), as well as sensors for measuring the pressure (5a, 5b) and the flow (4a, 4b) for activating of the control valve (3a, 3b), **characterised in that** the control valve is a valve according to one of claims 1 to 10.

12. The ventilation apparatus according to claim 11, **characterised in that** the air source (2) is configured as a rotating fan which maintains a constant rotational speed throughout a complete breathing cycle.

## Revendications

1. Soupape destinée à contrôler et à réguler le débit d'un appareil respiratoire (1), la soupape comportant un corps (11) et un registre rotatif (12) placé dans ce dernier et en étant rotatif autour de son axe longitudinal, par rotation du registre rotatif (12) au moins un orifice de passage (11d) pouvant être fermé en totalité ou en partie, le registre rotatif (12) comportant une surface d'étanchéité (12c) conique, qui interagit avec une surface d'appui (11f) correspondante du corps (11) et l'orifice de passage (11d) étant placé dans la région de la surface d'étanchéité (11f), **caractérisée en ce que** le registre rotatif (12) est appuyé en direction axiale sur le corps (11) par l'intermédiaire d'un appui ponctuel central, de préférence une bille (13) placée dans l'axe médian longitudinal.

2. Soupape selon la revendication 1, **caractérisée en ce que** le cône de la surface d'étanchéité (12c) est conçu en se rétrécissant à l'encontre d'une direction de débit.

3. Soupape selon la revendication 1 ou 2, **caractérisée en ce que** l'angle de conicité de la surface d'étanchéité est compris entre 60° et 120°, étant de préférence d'environ 90°.

4. Soupape selon l'une quelconque des revendications précédentes, **caractérisée en ce que** l'orifice de passage (11d) est conçu de telle sorte que, lors d'une rotation du registre rotatif (12) dans la direction de fermeture, la section transversale de débit rétrécisse progressivement et/ou **en ce que** plusieurs orifices de passage sont prévus.

5. Soupape selon la revendication 4, **caractérisée en ce que** l'orifice de passage ou les orifices de passages (11d) est (sont) conçu(s) en se rétrécissant de manière cunéiforme dans la direction de fermeture du registre rotatif (12).

6. Soupape selon l'une quelconque des revendications précédentes, **caractérisée en ce que** le rayon de conicité (r/R) des surfaces d'étanchéité du corps (31) interagissant mutuellement et/ou du registre rotatif (32) s'agrandit en continu sur des parties de la périphérie, à l'encontre de la direction de fermeture de la soupape.

7. Soupape selon l'une quelconque des revendications précédentes, **caractérisée en ce que** sur le côté opposé au registre rotatif (12), les orifices de passage (11d) du corps (11) sont entourés d'un bourrelet (11g), la hauteur du bord du bourrelet (11g) croissant vers la position de fermeture de la soupape.

8. Soupape selon l'une quelconque des revendications précédentes, **caractérisée en ce que** le registre rotatif (12) est proportionnellement activé par un actionneur, de préférence par un moteur pas-à-pas (17, 37).

9. Soupape selon la revendication 8, **caractérisée en ce qu'**entre le registre rotatif (12) et l'actionneur est placé un accouplement (38) élastique amovible.

10. Soupape selon l'une quelconque des revendications précédentes, **caractérisée en ce que** le registre rotatif (12, 32) est rotatif contre la force de rappel d'au moins un ressort à torsion (14, 34).

11. Respirateur pour la respiration artificielle ou pour l'assistance de la respiration artificielle d'un patient, avec une entrée d'air (1), une source d'air (2), au moins une soupape de commande (3a, 3b) à régulation proportionnelle, ainsi que des capteurs pour mesurer la pression (5a, 5b) et le débit (4a, 4b) pour activer la soupape de commande (3a, 3b), **caractérisé en ce que** la soupape de commande est une soupape selon l'une quelconque des revendications 1 à 10.

12. Respirateur selon la revendication 11, **caractérisé en ce que** la source d'air (2) est conçue sous la forme d'une ventilateur tournant à une vitesse de rotation constante sur d'un cycle respiratoire entier.
